(19) Europäisches Patentamt
European Patent Office
Office européen des brevets

(11) Veröffentlichungsnummer: **0 067 347**
**A1**

# (12) EUROPÄISCHE PATENTANMELDUNG

(21) Anmeldenummer: **82104732.1**

(22) Anmeldetag: **28.05.82**

(51) Int. Cl.³: **A 61 K 31/415**
//(A61K31/415, 31/215)

(30) Priorität: **11.06.81 CH 3835/81**
**22.02.82 CH 1080/82**

(43) Veröffentlichungstag der Anmeldung:
**22.12.82 Patentblatt 82/51**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Klecak, Georg, Dr.**
**Rheinparkstrasse 5**
**CH-4127 Birsfelden(CH)**

(72) Erfinder: **Müller, Marcel, Dr.**
**Quellenweg 10**
**CH-4402 Frenkendorf(CH)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) **Pharmazeutische Präparate.**

(57) Topisch applizierbares pharmazeutisches Präparat, enthaltend ein D-Homosteroid der Formel

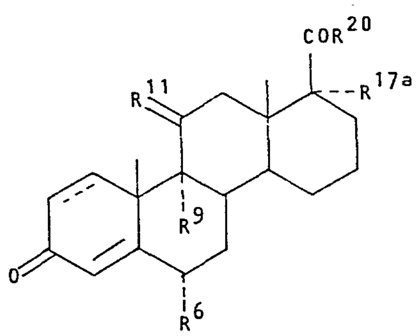

1

in der $R^6$ Wasserstoff, Methyl, Chlor oder Fluor, $R^9$ Wasserstoff, Fluor oder Chlor, $R^{11}$ Oxo oder (α–H, β–OH), $R^{17a}$ Hydroxy oder Acyloxy, $R^{20}$ eine Gruppe $-O-R^{201}$ oder $-CH_2-R^{21}$, $R^{201}$ nieder-Alkyl, Chlor-nieder-alkyl oder Fluor-nieder-alkyl, $R^{21}$ Wasserstoff, Chlor, Fluor, Hydroxy oder Acyloxy und die punktierte 1,2-Bindung eine fakultative C–C–Bindung bedeuten, und, falls $R^{20}$ eine Gruppe $-O-R^{201}$ ist, eine Doppelbindung in 16,17-Stellung vorliegen kann,
und eine Verbindung der Formel

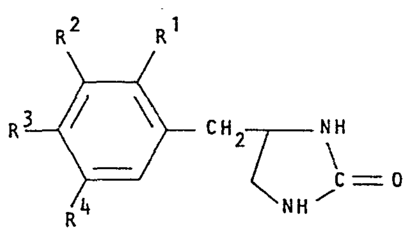

11

in der $R^1$ Wasserstoff, Halogen, nieder-Alkyl, nieder-Alkoxy und $R^2$, $R^3$ und $R^4$ Wasserstoff, nieder-Alkoxy, Hydroxy-nieder-alkoxy, oder zwei benachbarte Reste $R^1$, $R^2$, $R^3$ und $R^4$ zusammem Methylendioxy bedeuten, und mindestens ein Rest $R^1$, $R^2$, $R^3$ oder $R^4$ Sauerstoff enthält.

EP 0 067 347 A1

F.Hoffmann-La Roche & Co.Aktiengesellschaft, Basel/Schweiz

RAN 4051/9

# Pharmazeutische Präparate

Die vorliegende Erfindung betrifft topisch applizierbare pharmazeutische Präparate, mit insbesondere antiinflammatorischer Wirkung. Die erfindungsgemässen Präparate enthalten als Wirkstoffe ein D-Homosteroid der Formel

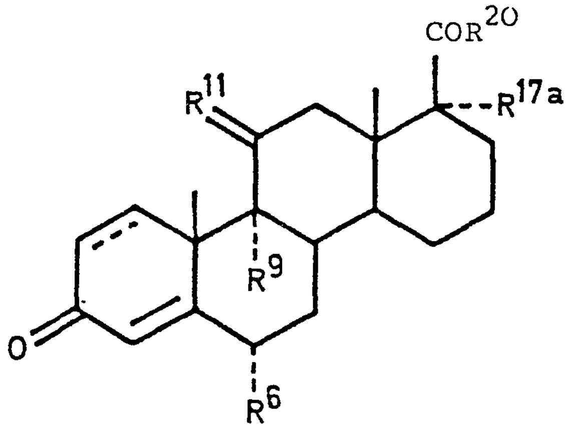

I

in der $R^6$ Wasserstoff, Methyl, Chlor oder Fluor, $R^9$ Wasserstoff, Fluor oder Chlor, $R^{11}$ Oxo oder (α-H,β-OH), $R^{17a}$ Hydroxy oder Acyloxy, $R^{20}$ eine Gruppe $-O-R^{201}$ oder $-CH_2-R^{21}$, $R^{201}$ nieder-Alkyl, Chlor-nieder-alkyl oder Fluor-nieder-alkyl, $R^{21}$ Wasserstoff, Chlor, Fluor, Hydroxy oder Acyloxy und die punktierte 1,2-Bindung eine fakultative C-C-Bindung bedeuten, und, falls $R^{20}$ eine Gruppe-$O-R^{201}$ ist, eine Doppelbindung in 16,17-Stellung vorliegen kann,

Mé/19.2.82

und eine Verbindung der Formel

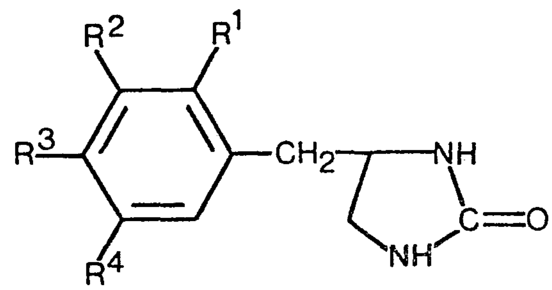

II

in der $R^1$ Wasserstoff, Halogen, nieder-Alkyl, nieder-Alkoxy und $R^2$, $R^3$ und $R^4$ Wasserstoff, nieder-Alkoxy, Hydroxy-nieder-alkoxy, oder zwei benachbarte Reste $R^1$, $R^2$, $R^3$ und $R^4$ zusammen Methylendioxy bedeuten, und mindestens ein Rest $R^1$, $R^2$, $R^3$ oder $R^4$ Sauerstoff enthält.

Eine Acyloxygruppe kann sich von einer gesättigten oder ungesättigten aliphatischen Carbonsäure mit bis zu 7 C-Atomen, wie Ameisensäure, Essigsäure, Propionsäure, Buttersäure, Pivalinsäure, Valeriansäure und Oenanthsäure, ableiten. Bevorzugt sind $C_{1-7}$-Alkanoyloxygruppen, wie Acetoxy, Propionyloxy und Butyryloxy. Nieder-alkylgruppen können geradkettig oder verzweigt sein und 1 bis 6, vorzugsweise 1 bis 4 C-Atome, enthalten. Beispiele solcher Gruppen sind Methyl, Aethyl, Propyl und n-Butyl.

Bevorzugte D-Homosteroide der Formel I sind diejenigen, worin das D-Homosteroid der Formel I in 1,2-Stellung ungesättigt ist und $R^6$ Wasserstoff, $R^9$ Wasserstoff oder Fluor, $R^{11}$ Hydroxy, $R^{17a}$ Acyloxy, insbesondere $C_{1-7}$-Alkanoyloxy, $R^{20}$ eine Gruppe $-CH_2-R^{21}$ und $R^{21}$ Acyloxy, insbesondere $C_{1-7}$-Alkanoyloxy, oder Wasserstoff oder Hydroxy ist. Besonders bevorzugt sind die 1,2-ungesättigten D-Homosteroide der Formel I, worin $R^6$ und $R^9$ Wasserstoff, $R^{11}$ Hydroxy, $R^{17a}$ Propionyloxy oder insbesondere Butyryloxy, $R^{20}$ eine Gruppe $-CH_2-R^{21}$ und $R^{21}$ Acetoxy, Hydroxy oder insbesondere Wasserstoff sind. Beispiele von bevorzugten Verbindungen sind:

17a-Butyryloxy-9-fluor-11β-hydroxy-D-homopregna-1,4-dien-3,20-dion;

21-Acetoxy-9-fluor-11β-hydroxy-17a-propionyloxy-D-homopregna-1,4-dien-3,20-dion;

17a-Butyryloxy-11β,21-dihydroxy-D-homopregna-1,4-dien-3,20-dion; und insbesondere

17a-Butyryloxy-11β-hydroxy-D-homopregna-1,4-dien-3,20-dion.

Beispiele von Verbindungen der Formel I sind ferner:

21-Chlor-11β,17a-dihydroxy-9-fluor-D-homopregna-1,4-dien-3,20-dion;

17a-Butyryloxy-21-chlor-9-fluor-11β-hydroxy-D-homopregna-1,4-dien-3,20-dion;

17a,21-Diacetoxy-9-fluor-11β-hydroxy-D-homopregna-1,4-dien-3,20-dion;

17a-Butyryloxy-11β,21-dihydroxy-9-fluor-D-homopregna-1,4-dien-3,20-dion;

9-Chlor-17a,21-dihydroxy-11β-hydroxy-D-homopregna-1,4-dien-3,20-dion;

21-Acetoxy-9-fluor-11β-hydroxy-17a-propionyloxy-D-homopregna-4-en-3,20-dion;

17a-Butyryloxy-11β-hydroxy-D-homopregna-4-en-3,20-dion;

11β-Hydroxy-3-oxo-17a-propionyloxy-D-homoandrosta-1,4-dien-17aβ-carbonsäure-methylester;

11β-Hydroxy-3-oxo-17a-propionyloxy-D-homoandrosta-1,4-dien-17aβ-carbonsäure-chlormethylester;

17a-Acetoxy-6α,9-difluor-11β-hydroxy-3-oxo-D-homoandrosta-1,4-dien-17aβ-carbonsäure-methylester.

Im Rahmen der vorliegenden Erfindung bevorzugte Verbindungen der Formel II sind diejenigen, worin $R^1$ und $R^4$ Wasserstoff, $R^2$ Aethoxy, Isopropoxy oder insbesondere Butoxy und $R^3$ Methoxy sind, insbesondere das D,L-4-(3-Butoxy-4-methoxybenzyl)-2-imidazolidinon.

Verbindungen der Formel II sind beispielsweise in der

deutschen Offenlegungsschrift 2 108 438 beschrieben. Ferner ist beispielsweise aus den deutschen Offenlegungsschriften 2 614 079 und 2 738 363 und der europäischen Offenlegungsschrift 13 959 bekannt, dass D-Homosteroide eine antiinflammatorische Wirkung haben. Es wurde nun gefunden, dass diese Wirkung durch Verbindungen der Formel II in überraschender Weise verstärkt wird.

Die erfindungsgemässen Präparate können zur Therapie von entzündlichen und allergischen dermatologischen Affektionen, wie Psoriasis, Eczemata, z.B. chronischen Eczemata, Dermatitis, z.B. Kontaktdermatitis und Neurodermatitis, und verwandten Erkrankungen, Verwendung finden. Die Erfindung betrifft die Verwendung der genannten Präparate bei der Behandlung von entzündlichen und allergischen dermatologischen Affektionen, sowie ein Verfahren zur Behandlung solcher Affektionen durch Verabreichung der erfindungsgemässen Präparate.

Beispiele topischer Applikationsformen der erfindungsgemässen Präparate sind Fettsalben, Salben, Crèmes, hydrophile Crèmes, Gels und Lotionen.

Zweckmässig beträgt die Konzentration an einem D-Homosteroid der Formel I zwischen etwa 0,001 und 0,5%, vorzugsweise zwischen etwa 0,005 und 0,05%, und die Konzentration an einer Verbindung der Formel II zwischen etwa 0,5 und 10%, vorzugsweise zwischen etwa 2 und 5%.

Die erfindungsgemässen Präparate können ein oder mehrere D-Homosteroide der Formel I bzw. eine oder mehrere Verbindungen der Formel II enthalten.

Die Herstellung der erfindungsgemässen Präparate kann in jedem Fachmann geläufiger Weise durch Vermischen der Wirkstoffkomponenten mit geeigneten, nicht-toxischen, inerten, verträglichen festen oder flüssigen Trägermaterialien, einschliesslich der üblichen Hilfsmittel, wie Stabilisierungs-, Konservierungs-, Netz- oder Emulgiermitteln, erfolgen.

## Beispiel 1

Eine Salbe mit folgender Zusammensetzung wird in an sich bekannter Weise hergestellt:

| | | |
|---|---|---|
| 17a-Butyryloxy-11β-hydroxy-D-homopregna-1,4-dien-3,20-dion * | | 0,05 g oder 0,01 g |
| D,L-4-(3-Butoxy-4-methoxy-benzyl)-2-imidazolidinon * | | 5 g |
| Vaseline, weiss | | 35 g |
| Wachs, weiss | | 4 g |
| Paraffinöl, dickflüssig | | 18,995 g oder 18,990 g |
| DEHYMULS E ** | | 7 g |
| Wasser, entsalzt | ad | 100 g |

* feingemahlen

** höhermolekularer aliphatischer Mischester; Lieferant: Deutsche Hydrierwerke

## Beispiel 2

Eine Salbe mit folgender Zusammensetzung wird in an sich bekannter Weise hergestellt:

| | | |
|---|---|---|
| 17a-Butyryloxy-9-fluor-11β-hydroxy-D-homopregna-1,4-dien-3,20-dion | | 0,01 g oder 0,005 g |
| D,L-4-(3-Butoxy-4-methoxy-benzyl)-2-imidazolidinon | | 2,5 g |
| Vaseline, weiss | | 35 g |
| Wachs, weiss | | 5 g |
| Paraffinöl, dickflüssig | | 19 g |
| DEHYMULS E | | 7 g |
| Wasser, entsalzt | ad | 100 g |

Beispiel 3

Eine Fettsalbe mit folgender Zusammensetzung wird in an sich bekannter Weise hergestellt:

| | | |
|---|---|---|
| 17a-Butyryloxy-11β,21-dihydroxy-D-hompregna-1,4-dien-3,20,dion | 0,01 | g oder 0,05 g |
| D,L-4-(3-Butoxy-4-methoxy-benzyl)-2-imidazolidinon | 2,5 | g |
| Paraffinöl | 10 | g |
| Wachs, microkristallin | 15 | g |
| Vaseline | 72,5 | g |

Beispiel 4

Eine Créme mit folgender Zusammensetzung wird in an sich bekannter Weise hergestellt:

| | | |
|---|---|---|
| 21-Acetoxy-9-fluor-11β-hydroxy-17a-propionyloxy-D-homopregna-1,4-dien-3,20-dion | 0,01 | g oder 0,02 g |
| D,L-4-(3-Butoxy-4-methoxy-benzyl)-2-imidazolidinon | 4,0 | g |
| Glycerinmonostearat | 10,0 | g |
| Tween 60 * | 2,0 | g |
| Cetylalkohol | 5,0 | g |
| Paraffinöl, dickflüssig | 7,0 | g |
| Methyl Paraben ** | 0,15 | g |
| Propylenglykol | 20,0 | g |
| Wasser, entsalzt | ad 100 | g |

* Polyäthylenoxyd-sorbitanstearat

** 4-Hydroxybenzoesäuremethylester

Beispiel 5

Mit 0,01 g oder 0,03 g 17a-Butyryloxy-21-chlor-9-fluor-11β-hydroxy-D-homopregna-1,4-dien-3,20-dion als Wirkstoffkomponent der Formel I, 3,0 g D,L-4-(3-Isopropoxy-4-methoxybenzyl-2-imidazolidinon (micronisiert) als Wirkstoffkomponent der Formel II und den gleichen Trägern und Excipientien wie in den Beispielen 1-4 werden in an sich bekannter Weise diesen Beispielen entsprechende Präparate hergestellt.

## Patentansprüche

1. Topisch applizierbares pharmazeutisches Präparat, enthaltend ein D-Homosteroid der Formel

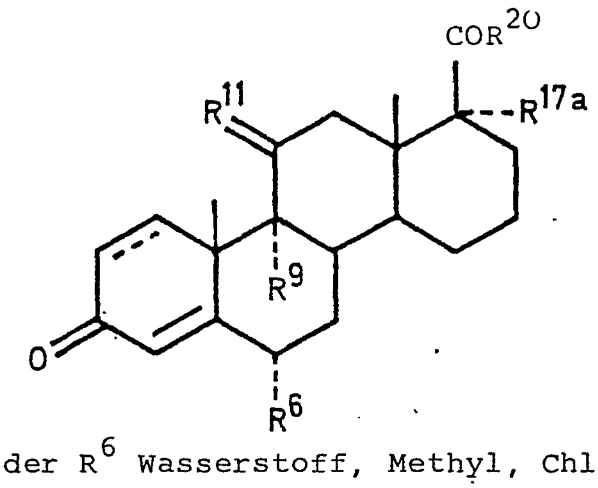

I

in der $R^6$ Wasserstoff, Methyl, Chlor oder Fluor, $R^9$ Wasserstoff, Fluor oder Chlor, $R^{11}$ Oxo oder (α-H,β-OH), $R^{17a}$ Hydroxy oder Acyloxy, $R^{20}$ eine Gruppe $-O-R^{201}$ oder $-CH_2-R^{21}$, $R^{201}$ nieder-Alkyl, Chlor-nieder-alkyl oder Fluor-nieder-alkyl, $R^{21}$ Wasserstoff, Chlor, Fluor, Hydroxy oder Acyloxy und die punktierte 1,2-Bindung eine fakultative C-C-Bindung bedeuten, und, falls $R^{20}$ eine Gruppe $-O-R^{201}$ ist, eine Doppelbindung in 16,17-Stellung vorliegen kann,

und eine Verbindung der Formel

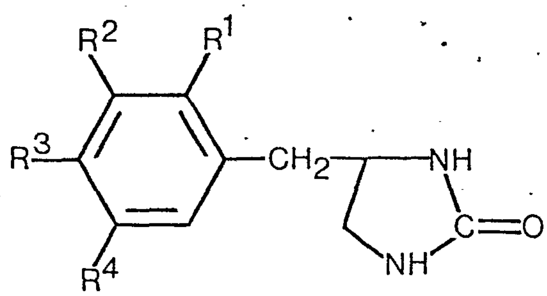

II

in der $R^1$ Wasserstoff, Halogen, nieder-Alkyl, nieder-Alkoxy und $R^2$, $R^3$ und $R^4$ Wasserstoff, nieder-Alkoxy, Hydroxy-nieder-alkoxy, oder zwei benachbarte Reste $R^1$, $R^2$, $R^3$ und $R^4$ zusammen Methylendioxy bedeuten, und mindestens ein Rest $R^1$, $R^2$, $R^3$ oder $R^4$ Sauerstoff enthält.

2. Präparat gemäss Anspruch 1, wobei das D-Homosteroid der Formel I in 1,2-Stellung ungesättigt ist und $R^6$ Wasserstoff, $R^9$ Wasserstoff oder Fluor, $R^{11}$ Hydroxy, $R^{17a}$ Acyloxy, insbesondere $C_{1-7}$-Alkanoyloxy, $R^{20}$ eine Gruppe $-CH_2-R^{21}$ und $R^{21}$ Acyloxy, insbesondere $C_{1-7}$-Alkanoyloxy, oder Wasserstoff oder Hydroxy ist.

3. Präparat gemäss Anspruch 1 oder 2, wobei im D-Homosteroid der Formel I eine Doppelbindung in 1,2-Stellung vorliegt und $R^6$ und $R^9$ Wasserstoff, $R^{11}$ Hydroxy, $R^{17a}$ Propionyloxy oder insbesondere Butyryloxy, $R^{20}$ eine Gruppe $-CH_2-R^{21}$ und $R^{21}$ Acetoxy, Hydroxy oder insbesondere Wasserstoff sind.

4. Präparat gemäss einem der Ansprüche 1-3, worin das D-Homosteroid das 17a-Butyryloxy-11β-hydroxy-D-homopregna-1,4-dien-3,20-dion ist.

5. Präparat gemäss einem der Ansprüche 1-4, wobei in der Verbindung der Formel II $R^1$ und $R^4$ Wasserstoff, $R^2$ Aethoxy, Isopropoxy oder insbesondere Butoxy und $R^3$ Methoxy sind.

6. Präparat gemäss einem der Ansprüche 1-5, worin die Verbindung der Formel II das D,L-4-(3-Butoxy-4-methoxybenzyl)-2-imidazolidinon ist.

7. Präparat gemäss einem der Ansprüche 1-6, worin die Verbindung I das 17a-Butyryloxy-11β-hydroxy-D-homopregna-1,4-dien-3,20-dion, das 17a-Butyryloxy-9-fluor-11β-hydroxy-D-homopregna-1,4-dien-3,20-dion, das 21-Acetoxy-9-fluor-11β-hydroxy-17a-propionyloxy-D-homopregna-1,4-dien-3,20-dion, das 17a-Butyryloxy-11β,21-dihydroxy-D-homopregna-1,4-dien-3,20-dion oder das 17a-Butyryloxy-21-chlor-9-fluor-11β-hydroxy-D-homopregna-1,4-dien-3,20-dion und die Verbindung der Formel II das D,L-4-(3-Butoxy-4-methoxybenzyl)-2-imidazolidinon oder das D,L-4-(3-Isopropoxy-4-methoxybenzyl)-2-imidazolidinon ist.

8. Präparat gemäss einem der Ansprüche 1-7, worin das D-Homosteroid das 17a-Butyryloxy-11β-hydroxy-D-homopregna-1,4-dien-3,20-dion und die Verbindung der Formel II das D,L-4-(3-Butoxy-4-methoxybenzyl)-2-imidazolidinon ist.

***

<u>Patentansprüche für Oesterreich</u>

1. Verfahren zur Herstellung von topisch applizierbaren pharmazeutischen Präparaten, dadurch gekennzeichnet, dass man ein D-Homosteroid der Formel

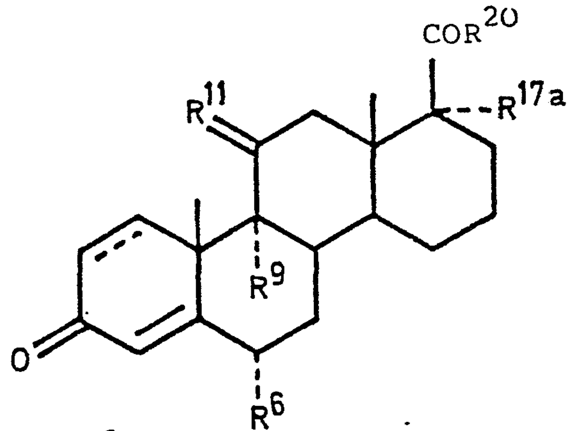

I

in der $R^6$ Wasserstoff, Methyl, Chlor oder Fluor, $R^9$ Wasserstoff, Fluor oder Chlor, $R^{11}$ Oxo oder (α-H,β-OH), $R^{17a}$ Hydroxy oder Acyloxy, $R^{20}$ eine Gruppe $-O-R^{201}$ oder $-CH_2-R^{21}$, $R^{201}$ nieder-Alkyl, Chlor-nieder-alkyl oder Fluor-nieder-alkyl, $R^{21}$ Wasserstoff, Chlor, Fluor, Hydroxy oder Acyloxy und die punktierte 1,2-Bindung eine fakultative C-C-Bindung bedeuten, und, falls $R^{20}$ eine Gruppe $-O-R^{201}$ ist, eine Doppelbindung in 16,17-Stellung vorliegen kann,

und eine Verbindung der Formel

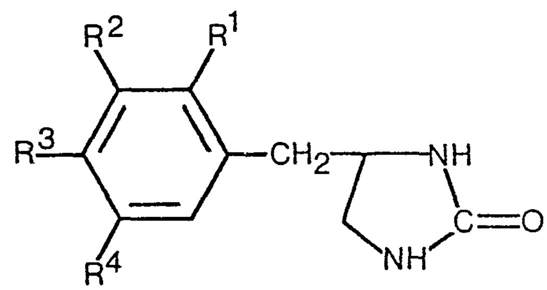

II

in der $R^1$ Wasserstoff, Halogen, nieder-Alkyl, nieder-Alkoxy und $R^2$, $R^3$ und $R^4$ Wasserstoff, nieder-Alkoxy, Hydroxy-nieder-alkoxy, oder zwei benachbarte Reste $R^1$, $R^2$, $R^3$ und $R^4$ zusammen Methylendioxy bedeuten, und mindestens ein Rest $R^1$, $R^2$, $R^3$ oder $R^4$ Sauerstoff enthält,

in Gegenwart nicht-toxischer, inerter, therapeutisch verträglicher Trägermaterialien miteinander vermischt und das Gemisch in eine geeignete topisch applizierbare galenische Form bringt.

2. Verfahren nach Anspruch 1, wobei das D-Homosteroid der Formel I in 1,2-Stellung ungesättigt ist und $R^6$ Wasserstoff, $R^9$ Wasserstoff oder Fluor, $R^{11}$ Hydroxy, $R^{17a}$ Acyloxy, insbesondere $C_{1-7}$-Alkanoyloxy, $R^{20}$ eine Gruppe $-CH_2-R^{21}$ und $R^{21}$ Acyloxy, insbesondere $C_{1-7}$-Alkanoyloxy, oder Wasserstoff oder Hydroxy ist.

3. Verfahren nach Anspruch 1 oder 2, wobei im D-Homosteroid der Formel I eine Doppelbindung in 1,2-Stellung vorliegt und $R^6$ und $R^9$ Wasserstoff, $R^{11}$ Hydroxy, $R^{17a}$ Propionyloxy oder insbesondere Butyryloxy, $R^{20}$ eine Gruppe $-CH_2-R^{21}$ und $R^{21}$ Acetoxy, Hydroxy oder insbesondere Wasserstoff sind.

4. Verfahren nach einem der Ansprüche 1-3, worin das D-Homosteroid das 17a-Butyryloxy-11β-hydroxy-D-homopregna-1,4-dien-3,20-dion ist.

5. Verfahren nach einem der Ansprüche 1-4, wobei in der Verbindung der Formel II $R^1$ und $R^4$ Wasserstoff, $R^2$ Aethoxy, Isopropoxy oder insbesondere Butoxy und $R^3$ Methoxy sind.

6. Verfahren nach einem der Ansprüche 1-5, wobei die Verbindung der Formel II das D,L-4-(3-Butoxy-4-methoxybenzyl)-2-imidazolidinon ist.

7. Verfahren nach einem der Ansprüche 1-6, wobei die Verbindung I das 17a-Butyryloxy-11β-hydroxy-D-homopregna-1,4-dien-3,20-dion, das 17a-Butyryloxy-9-fluor-11β-hydroxy-D-homopregna-1,4-dien-3,20-dion, das 21-Acetoxy-9-fluor-11β-hydroxy-17a-propionyloxy-D-homopregna-1,4-dien-3,20-dion, das 17a-Butyryloxy-11β-21-dihydroxy-D-homopregna-1,4-dien-3,20-dion oder das 17a-Butyryloxy-21-chlor-9-fluor-11β-hydroxy-D-homopregna-1,4-dien-3,20-dion und die Verbindung der Formel II das D,L-4-(3-Butoxy-4-methoxybenzyl)-2-imidazolidinon oder das D,L-4-(3-Isopropoxy-4-methoxybenzyl)-2-imidazolidinon ist.

8. Verfahren nach einem der Ansprüche 1-7, wobei das D-Homosteroid das 17a-Butyryloxy-11β-hydroxy-D-homopregna-1,4-dien-3,20-dion und die Verbindung der Formel II das D,L-4-(3-Butoxy-4-methoxybenzyl)-2-imidazolidinon ist.

***

0067347

Europäisches Patentamt

# EUROPÄISCHER RECHERCHENBERICHT

Nummer der Anmeldung

EP 82 10 4732

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-4 034 087 (JOHN J.VOORHEES) *Spalte 21, Zeilen 25-65; Ansprüche 1-4* & DE - A - 2 401 446<br>--- | 1-8 | A 61 K 31/415//<br>(A 61 K 31/415<br>A 61 K 31/215) |
| A | US-A-4 141 976 (JOHN J.VOORHEES) *Spalte 19, Zeilen 1-32*<br>--- | 1-8 | |
| D,A | DE-A-2 108 438 (F.HOFFMANN-LA ROCHE & CO.)<br>--- | 1-8 | |
| D,A | DE-A-2 614 079 (F.HOFFMANN-LA ROCHE & CO.)<br>--- | 1-8 | |
| D,A | DE-A-2 738 363 (F.HOFFMANN-LA ROCHE & CO.)<br>--- | 1-8 | RECHERCHIERTE SACHGEBIETE (Int. Cl. ³) |
| D,A | EP-A-0 013 959 (F.HOFFMANN-LA ROCHE & CO.)<br>----- | 1-8 | A 61 K |

Der vorliegende Recherchenbericht wurde für alle Patentansprüche erstellt.

| Recherchenort | Abschlußdatum der Recherche | Prüfer |
|---|---|---|
| DEN HAAG | 30-08-1982 | BRINKMANN C. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument